(19) Europäisches Patentamt
European Patent Office
Office européen des brevets

(11) Veröffentlichungsnummer: **0 030 916**
**B1**

# (12) EUROPÄISCHE PATENTSCHRIFT

(45) Veröffentlichungstag der Patentschrift: **29.08.84**

(51) Int. Cl.³: **C 07 D 491/044**, A 61 K 31/55 // (C07D491/044, 223/00, 313/00)

(21) Anmeldenummer: **80810377.4**

(22) Anmeldetag: **04.12.80**

(54) **Azatetracyclische Carbonitrile.**

(30) Priorität: **10.12.79 CH 10928/79**

(43) Veröffentlichungstag der Anmeldung: **24.06.81 Patentblatt 81/25**

(45) Bekanntmachung des Hinweises auf die Patenterteilung: **29.08.84 Patentblatt 84/35**

(84) Benannte Vertragsstaaten: **AT BE CH DE FR IT LI LU NL SE**

(56) Entgegenhaltungen:
**CH - A - 592 095**
**DE - A - 2 723 105**
**DE - A - 2 723 209**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Blattner, Hans, Burgstrasse 116, CH-4125 Riehen (CH)**
Erfinder: **Storni, Angelo, Dr., Im Feuerbusch 3, CH-4310 Rheinfelden (CH)**

EP 0 030 916 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue azatetracyclische Carbonitrile und ihre Säureadditionssalze mit wertvollen pharmakologischen Eigenschaften, Verfahren zu ihrer Herstellung, sowie pharmazeutische Zusammensetzungen, welche die neuen Stoffe als Wirksubstanzen enthalten, und deren Anwendung.

Die erfindungsgemäßen azatetracyclischen Carbonitrile entsprechen der Formel

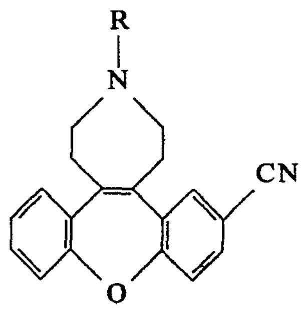

(I)

in welcher
R Wasserstoff, Niederalkyl mit bis zu 8 Kohlenstoffatomen, Cycloalkylniederalkyl mit bis zu 10 Kohlenstoffatomen, Allyl, 2-Methallyl, Propargyl, Hydroxyniederalkyl mit 2 bis 8 Kohlenstoffatomen, Niederalkoxyniederalkyl mit 3 bis 10 Kohlenstoffatomen oder Alkanoyloxyniederalkyl mit 3 bis 21 Kohlenstoffatomen bedeutet, wobei ein allfällig vorhandenes Sauerstoffatom durch mindestens 2 Kohlenstoffatome vom Ringstickstoffatom getrennt ist, sowie Säureadditionssalze von solchen Verbindungen.

Ebenfalls Gegenstand der Erfindung sind die Säureadditionssalze der Verbindungen der Formel I, insbesondere die pharmazeutisch annehmbaren Säureadditionssalze.

In der obigen Definition der Formel I wie auch nachstehend werden unter niederen Resten solche mit höchstens 8 und vorzugsweise höchstens 4 Kohlenstoffatomen verstanden.

R enthält als Niederalkyl vorzugsweise 1 bis 6 Kohlenstoffatome. Diese Niederalkylgruppen, die geradkettig oder verzweigt sein können, sind z. B. Methyl, Äthyl, Propyl, Isopropyl, Butyl, Isobutyl oder Tertiärbutyl.

Als Cycloalkylniederalkyl enthält der Rest R vorzugsweise 4 bis 8 Kohlenstoffatome. Cycloalkylniederalkyl ist also z. B. Cyclopropylmethyl, Cyclobutylmethyl und insbesondere Cyclopentylmethyl, Cyclohexylmethyl, weiter z. B. Cyclopropyläthyl, Cyclobutyläthyl, Cyclopentyläthyl, Cyclohexyläthyl.

Im Hydroxyniederalkyl als Rest R ist die Hydroxylgruppe durch mindestens 2 Kohlenstoffatome vom Ringstickstoffatom getrennt. Dieser Rest enthält 2 bis 8 und bevorzugt 2 bis 4 Kohlenstoffatome. Das Hydroxyniederalkyl, das geradkettig oder verzweigt sein kann, ist z. B. das 1-Methyl-2-hydroxyäthyl, 2-Hydroxypropyl, 1- oder 2-Methyl-2-hydroxypropyl, und insbesondere das 2-Hydroxy-äthyl und das 3-Hydroxypropyl.

Im Niederalkoxyniederalkyl als Rest ist das Sauerstoffatom durch mindestens 2 Kohlenstoffatome vom Ringstockstoffatom getrennt. Dieser Rest enthält z. B. 3 bis 10 und bevorzugt 3 bis 6 Kohlenstoffatome. Dieses Niederalkoxyniederalkyl ist z. B. das 2-Methoxy-propyl, 2- oder 3-Äthoxypropyl, 2- oder 3-Propoxy-propyl, 3-Isopropoxypropyl und insbesondere das 2-Methoxy oder 2-Äthoxy-äthyl und vor allem das 3-Methoxy-propyl.

Im Alkanoyloxyniederalkyl als Rest ist das Ester-Sauerstoffatom durch mindestens 2 Kohlenstoffatome vom Ringstickstoffatom getrennt. Dieser Rest enthält z. B. 3 bis 21 und bevorzugt 4 bis 11 Kohlenstoffatome. Dieser Rest ist z. B. 2-Formyloxy-äthyl, 2-Acetyloxy-äthyl, 2-Propionyloxy-äthyl, 2-Acetyloxy-propyl, 2-Methyl-3-acetyloxy-propyl oder 2- oder 3- Propionyloxy-propyl und insbesondere 3-Acetyloxy-propyl und 3-Oktanoyloxy-propyl.

Salze von Verbindungen der Formel I sind in erster Linie Säureadditionssalze, insbesondere pharmazeutisch annehmbare Säureadditionssalze, z. B. mit anorgansichen Säuren, wie Chlorwasserstoffsäure, Bromwasserstoffsäure, Schwefelsäure oder Phosphorsäure, oder mit organischen Säuren, wie organischen Carbon- und Sulfonsäuren, wie Methansulfonsäure, Äthansulfonsäure, 2-Hydroxyäthansulfonsäure, Essigsäure, Äpfelsäure, Weinsäure, Zitronensäure, Milchsäure. Oxalsäure, Bernsteinsäure, Fumarsäure, Maleinsäure, Benzoesäure, Salicylsäure, Phenylessigsäure, Mandelsäure oder Embonsäure.

Die neuen azatetracyclischen Carbonitrile der allgemeinen Formel I und ihre Säureadditionssalze weisen wertvolle pharmakologische, z. B. auf das Zentralnervensystem wirkende, Eigenschaften auf. Sie zeichnen sich in erster Linie durch zentraldämpfende, erregungshemmende (Amphetamin-antagonistische) Wirkungen aus, was anhand von pharmakologischen Versuchen nachgewiesen werden kann. So zeigen sie an der Ratte im Amphetamin-Antagonismus-Test (Niemegeers und Hanssen, Arzneimittelforsch., Bd. 24, S. 45 (1974)) in einem Dosisbereich von 0,01 bis 1 mf/kg i. p. oder per os eine erregungshemmende Wirkung. Die neuen Verbindungen sind somit im Vergleich zu den strukturähnlichen, in DE-A 2 723 105 spezifisch beschriebenen Verbindungen in der Wirkung um ein Vielfaches

überlegen (s. Seite 16, letzter Absatz). Die kataleptische Wirkung ist gegenüber der Amphetamin-antagonistischen Wirkung verhältnismäßig gering. Die neuen azatetracyclischen Carbonitrile der allgemeinen Formel I und ihre pharmazeutisch annehmbaren Säureadditionssalze können deshalb als tranquillisierende, antipsychotische und erregungshemmende Verbindungen zur Behandlung von Erregungszuständen verschiedener Genese verwendet werden.

Die Erfindung betrifft insbesondere Verbindungen der Formel I, worin R Wasserstoff, Niederalkyl mit bis zu 4 Kohlenstoffatomen, z. B. Methyl oder Äthyl, Cycloalkylniederalkyl mit 4—8 Kohlenstoffatomen, z. B. Cyclopentylmethyl und Cyclohexylmethyl, Allyl, Propargyl, Hydroxyniederalkyl mit 2 bis 4 Kohlenstoffatomen, z. B. 2-Hydroxyäthyl und 3-Hydroxypropyl, Niederalkoxyniederalkyl mit 3 bis 6 Kohlenstoffatomen, z. B. 3-Methoxypropyl, oder Alkanoyloxy-niederalkyl mit 4 bis 11 Kohlenstoffatomen, z. B. Acetoxy-propyl und Oktanoyloxypropyl bedeuten und Salze, insbesondere Säureadditionssalze, in erster Linie pharmazeutisch annehmbare Säureadditionssalze davon.

Die Erfindung betrifft vor allem Verbindungen der Formel I, worin R Niederalkyl, z. B. Methyl oder Äthyl, oder Cyclopentylmethyl bedeutet, wie das 7-Cyano-3-methyl-2,3,4,5-tetrahydro-1H-dibenzo[2,3 : 6,7]azepin, oder das 3-(Cyclopentylmethyl)-7-cyano-2,3,4,5-tetrahydro-1H-dibenzo[2,3 : 6,7]oxepino[4,5-d]azepin, und Salze davon, insbesondere Säureadditionssalze, in erster Linie pharmazeutisch annehmbare Säureadditionssalze davon.

Die Verbindungen der Formel I werden in an sich bekannter Weise hergestellt. So erhält man sie z. B., indem man einen reaktionsfähigen Diester des Diäthanols der Formel

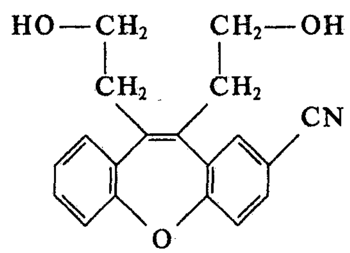

(II)

mit einer Verbindung der Formel

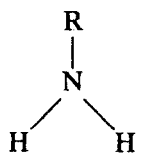

(III)

umsetzt.

Als reaktionsfähige Diester eines Diäthanols der Formel II können Ester von starken anorganischen Säuren, wie z. B. Bis-chlorwasserstoff, Bis-jodwasserstoffsäureester oder insbesondere Bis-bromwasserstoffsäureester oder Bromwasserstoff-chlorwasserstoffsäureester eingesetzt werden. Ferner können auch entsprechende Diester von starken organischen Säuren, z. B. von Sulfonsäuren, wie Methansulfonsäure, Benzolsulfonsäure, p-Chlor- oder p-Brombenzolsulfonsäure oder p-Toluolsulfonsäure, verwendet werden. Diese Diester der Verbindungen der Formel II werden vorzugsweise in einem geeigneten inerten Lösungsmittel bei einer Reaktionstemperatur von 20 bis 130°C umgesetzt. Als inerte Lösungsmittel eignen sich beispielsweise Kohlenwasserstoffe, wie Benzol oder Toluol, Halogenkohlenwasserstoffe, wie Chloroform, niedere Alkohole, wie Äthanol und insbesondere Methanol, ätherartige Flüssigkeiten, wie Äther oder Dioxan, sowie niedere Alkanone, z. B. Aceton, Methyläthylketon oder Diäthylketon oder Gemische von solchen Lösungsmitteln, z. B. Benzol—Methanol.

Bei der erfindungsgemäßen Umsetzung von einem Moläquivalent Diester eines Diäthanols der Formel II mit einem Moläquivalent freier Base der Formel III werden zwei Moläquivalente Säure abgespalten, die vorzugsweise an ein säurebindendes Mittel gebunden werden. Als säurebindende Mittel eignen sich z. B. Alkalicarbonate, wie Kaliumcarbonat, oder z. B. Alkalihydroxide, wie Natriumhydroxid oder Kaliumhydroxid, oder überschüssige Base der Formel III, ferner tertiäre organische Basen, wie Pyridin und insbesondere Triäthylamin oder N-Äthyl-di-isopropylamin.

Die unmittelbaren Ausgangsstoffe, also die reaktionsfähigen Diester der Formel II, können aus den entsprechenden Diäthanolen durch Veresterung bzw. Ersatz der Hydroxygruppen durch Halogen nach üblichen Methoden hergestellt werden. Die Diäthanole wiederum lassen sich aus den entsprechenden Diessigsäuremethylestern durch Reduktion mit Lithiumaluminiumhydrid herstellen. Die Diessigsäuremethylester können aus den entsprechenden Diacetonitrilen mit Methanol und 2 Moläquivalenten Wasser unter Einleiten von Chlorwasserstoff hergestellt werden. Die Diacetonitrile wiederum lassen sich aus den entsprechenden Bis-(6-brommethyl)-verbindungen mit Natriumcyanid gewinnen.

Nach einem weiteren Verfahren erhält man die Verbindungen der Formel I, indem man eine Verbindung der Formel

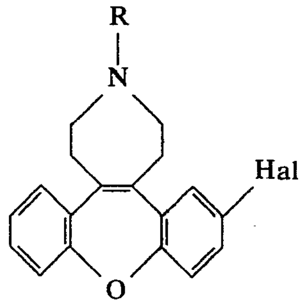

(IV)

in welcher Hal ein Halogenatom darstellt, mit einer Cyanidverbindung umsetzt.

Als Reste Hal in Verbindungen der Formel IV wählt man Chlor oder Jod, aber vorzugsweise Brom. Cyanidverbindungen sind in erster Linie Alkalimetall- oder Schwermetallcyanide. Als Alkalicyanid wird Natriumcyanid bevorzugt. Aber ganz besonders eignet sich Kupfer-I-cyanid, als Vertreter der Schwermetallcyanide. Die Reaktion kann in An- oder Abwesenheit von Lösungsmitteln durchgeführt werden, und dies in einem Temperaturbereich von 80 bis 250°C. Als Lösungsmittel eignen sich besonders Pyridin, Chinolin, Dimethylformamid, 1-Methyl-2-pyrrolidinon und Hexamethylphosphorsäuretriamid. Die beiden letztgenannten eignen sich besonders für Kupfer(I)-cyanid als Cyanierungsmittel.

Die Ausgangsstoffe der Formel IV sind bekannt oder können nach an sich bekannten Methoden, wie z. B. analog dem erstgenannten Verfahren, hergestellt werden. In diesem Zusammenhang wird u. a. auf DE-OS 2 723 105 verwiesen.

Anschließend an die erfindungsgemäßen Umsetzungen kann gegebenenfalls eine Reihe von Umwandlungen vorgenommen werden, die Verbindungen der Formel I in andere Verbindungen der Formel I überführen.

Gegebenenfalls kann eine Verbindung der Formel I, deren Rest R Wasserstoff darstellt, in ein Verfahrensprodukt übergeführt, werden, dessen Rest eine der anderen Bedeutungen hat.

So kann z. B. eine N-Substitution erfolgen, entweder mit einem reaktionsfähigen Ester eines entsprechenden Alkohols der Formel $R_1-OH$, worin $R_1$ die gleiche Bedeutung wie R in Formel I hat mit Ausnahme von Wasserstoff, oder durch eine Umsetzung mit entsprechenden Aldehyden oder Ketonen unter reduzierenden Bedingungen.

Die Umsetzung von Verbindungen der Formel I, in der R Wasserstoff bedeutet, mit einem reaktionsfähigen Ester einer Hydroxyverbindung der Formel $R_1-OH$ wird vorzugsweise in einem Lösungsmittel bei einer Reaktionstemperatur von 20 bis 130°C, insbesondere bei der Siedetemperatur des Lösungsmittels, vorgenommen.

Als reaktionsfähige Ester können z. B. Halogenide, wie Chloride oder Bromide, ferner aromatische oder aliphatische Sulfonsäureester, wie der p-Toluolsulfonsäure-methylester oder -äthylester, bzw. der Methansulfonsäure-methylester, -äthylester oder Cyclopentylmethylester oder Schwefelsäureester, wie z. B. das Dimethyl- oder Diäthylsulfat, verwendet werden. Als säurebindende Mittel eignen sich Alkalimetallcarbonate, wie z. B. Kaliumcarbonat, oder Alkalihydroxide, wie z. B. Natriumhydroxid, oder tertiäre, organische Basen, wie z. B. Pyridin oder das N-Äthyldiisopropylamin. Geeignete Lösungsmittel sind solche Lösungsmittel, die unter den Reaktionsbedingungen inert sind, beispielsweise Kohlenwasserstoffe, wie Benzol oder Toluol, ferner Alkanole, wie z. B. Methanol oder Äthanol, oder Alkanone, wie Aceton oder Methyläthylketon.

Aldehyde und Ketone, die den Alkoholen der Formel $R_1-OH$ entsprechen sind z. B. niedere aliphatische Aldehyde oder Ketone, niedere freie, veresterte oder verätherte Hydroxyoxoalkane oder veresterte Oxoalkancarbonsäuren. Das erhaltene Reaktionsprodukt bei der Umsetzung dieser Aldehyde oder Ketone mit den genannten Verbindungen der Formel I kann im gleichen Arbeitsgang oder anschließend reduziert werden.

Die Aldehyde, z. B. Formaldehyd oder Acetaldehyd, oder die Ketone, z. B. Aceton, werden beispielsweise mit den genannten Verbindungen der Formel I in einem inerten Lösungsmittel auf ca. 30° bis 100°C erwärmt, und gleichzeitig oder anschließend wird das Reaktionsgemisch mit Wasserstoff in Gegenwart eines Katalysators hydriert. Geeignete Lösungsmittel sind z. B. Alkanole, wie Methanol oder Äthanol, und geeignete Katalysatoren Edelmetallkatalysatoren, wie Palladium auf Kohle.

Anstelle von Wasserstoff in Gegenwart eines Katalyators können aber auch andere Reduktionsmittel, z. B. Ameisensäure, für die reduktive Alkylierung verwendet werden. Nach dieser Variante des Verfahrens werden die genannten Verbindungen der Formel I mit Ameisensäure und den genannten Typen von Aldehyden oder Ketonen, insbesondere Formaldehyd, vorzugsweise ohne Lösungsmittel, erwärmt.

Ferner kann gegebenenfalls eine Verbindung der Formel I, dessen Rest R eine Hydroxyniederalkylgruppe darstellt, zu einer Verbindung acyliert werden, deren Rest R eine veresterte Hydroxyniederalkylgruppe bedeutet.

Die Acylierung kann z. B. mit einem Carbonsäureanhydrid oder mit einem entsprechenden Carbonsäurehalogenid bei einer Reaktionstemperatur zwischen ca. 20 und 100°C vorgenommen werden. Da die Kondensation unter Abspaltung von Säure vor sich geht, ist es zweckmäßig, dem Reaktionsgemisch ein säurebindendes Mittel, z. B. eine tertiäre organische Base, wie Pyridin, beizufügen. Überschüssige tertiäre organische Base kann auch als Lösungsmittel verwendet werden. Ferner kann man als Lösungsmittel Kohlenwasserstoffe, z. B. Benzol oder Toluol, oder Halogenkohlenwasserstoffe, z. B. Chloroform, einsetzen.

Ferner kann gegebenenfalls eine Verbindung der Formel I, in der R von Wasserstoff verschieden ist in ein Verfahrensprodukt übergeführt werden, dessen Rest R Wasserstoff bedeutet. Dies erreicht man vorteilhafterweise dadurch, daß man diese Gruppe R, vorzugsweise eine leicht abspaltbare Gruppe wie die Methyl- oder Allylgruppe, durch einen durch Reduktion einschließlich Hydrogenolyse abspaltbaren Rest, insbesondere mittels Umsetzung mit einem Halogenameisensäure-2-arylalkylester oder 2-halogenalkylester, durch einen 2-Arylalkoxycarbonylrest, z. B. den Benzyloxycarbonylrest, oder einen 2-Halogen-alkoxycarbonylrest, z. B. den 2,2,2-Trichloräthoxycarbonylrest ersetzt und diesen Rest mittels Hydrogenolyse oder Reduktion in üblicher Weise durch Wasserstoff ersetzt. 2-Arylalkoxycarbonylreste können z. B. mittels Wasserstoff in Gegenwart eines Hydrierungskatalysators, beispielsweise Platin, Palladium oder Raney-Nickel, und gegebenenfalls von Chlorwasserstoff, bei Raumtemperatur und Normaldruck oder bei mäßig erhöhten Temperaturen und Drücken in geeigneten organischen Lösungsmittel, wie z. B. Methanol, Äthanol oder Dioxan weghydriert werden. 2-Halogen-alkoxycarbonylreste, wie neben dem 2,2,2-Trichloräthoxycarbonylrest, z. B. auch der 2-Jodäthoxy- oder 2,2,2-Tribromäthoxycarbonylrest, können insbesondere durch metallische Reduktion (sog. naszierenden Wasserstoff) entfernt werden. Naszierender Wasserstoff kann dabei durch Einwirkung von Metall oder Metall-Legierungen, wie Amalgamen, auf Wasserstoff liefernde Mittel, wie Carbonsäuren, Alkohole oder Wasser erhalten werden, wobei insbesondere Zink oder Zinklegierungen zusammen mit Essigsäure in Betracht kommen. Die reduktive Abspaltung von 2-Halogen-alkoxy-carbonylresten kann ferner durch Chrom(II)-verbindungen, wie Chrom(II)-chlorid oder Chrom(II)-acetat erfolgen.

Je nach den Verfahrensbedingungen und Ausgangsstoffen erhält man die Endstoffe gegebenenfalls in freier Form oder in Form ihrer Salze, die sich in üblicher Weise ineinander oder in andere Salze umwandeln lassen. So bildet man freie Verbindungen der Formel I aus erhaltenen Säureadditionssalzen, z. B. durch Behandeln mit Basen oder basischen Ionenaustauschern, während man freie Basen der Formel I z. B. durch Umsetzen mit organischen oder anorganischen Säuren, insbesondere solchen, die zur Bildung pharmazeutisch verwendbarer Salze geeignet sind, wie den obengenannten, in Säureadditionssalze überführt.

Salze der neuen Verbindungen können auch zu Reinigungszwecken verwendet werden, z. B. indem man die freien Verbindungen in ihre Salze überführt, diese isoliert und gegebenenfalls reinigt, und wieder in die freien Verbindungen überführt. Infolge der engen Beziehungen zwischen den neuen Verbindungen in freier Form und in Form ihrer Salze sind im Vorausgegangenen und nachfolgend unter den freien Verbindungen sinn- und zweckmäßig gegebenenfalls auch die entsprechenden Salze zu verstehen.

Erhaltene Racemate lassen sich nach an sich bekannten Methoden in die Antipoden zerlegen, z. B. durch Umkristallisieren aus einem optisch aktiven Lösungsmittel, durch Behandeln mit geeigneten Mikroorganismen oder durch Umsetzen mit einer, mit der racemischen Verbindung Salze bildenden optisch aktiven Substanz, insbesondere Säure, und Trennen des auf diese Weise erhaltenen Salzgemisches, z. B. auf Grund von verschiedenen Löslichkeiten, in die diastereomeren Salze, aus denen die freien Antipoden durch Einwirkung geeigneter Mittel freisetzt werden können. Besonders gebräuchliche, optisch aktive Säuren sind z. B. die D- und L-Formen von Weinsäure, Di-O,O'-p-toluoyl-weinsäure, Apfelsäure, Mandelsäure, Camphersulfonsäure, Glutaminsäure, Asparaginsäure oder Chinasäure. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder bei denen man einen Ausgangsstoff unter der Reaktionsbedingungen bildet oder bei denen eine Reaktionskomponente gegebenenfalls in Form ihrer Salze vorliegt.

Zweckmäßig verwendet man für die Durchführung des erfindungsgemäßen Verfahrens solche Ausgangsstoffe, die zu den eingangs besonders erwähnten Gruppen von Endstoffen und besonders zu den speziell beschriebenen oder hervorgehobenen Endstoffen führen.

Die neuen Verbindungen können z. B. in Form pharmazeutischer Präparate Verwendung finden, welche eine wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z. B. oralen, oder parenteralen Verabreichung eignen. So verwendet man Tabletten oder Gelatinekapseln, welche den Wirkstoff zusammen mit Verdünnungsmitteln, z. B. Laktose, Dextrose, Sukrose, Mannitol, Sorbitol, Cellulose und/oder Glycin, und Schmiermitteln, z. B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z. B. Magnesiumaluminiumsilikat, Stärken, wie Mais-, Weizen-, Reis- oder Pfeilwurzelstärke, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, wie Natriumalginat, und/oder Brausemischungen, oder Adsorptionsmittel, Farbstoffe, Geschmackstoffe und Süßmittel. Ferner kann man die neuen pharmakologisch

wirksamen Verbindungen in Form von injizierbaren, z. B. intravenös, verabreichbaren Präparaten oder von Infusionslösungen verwenden. Solche Lösungen sind vorzugsweise isotonische wäßrige Lösungen oder Suspensionen, wobei diese z. B. als lyophilisierten Präparate, welche die Wirksubstanz allein oder zusammen mit einem Trägermaterial, z. B. Mannit, enthalten, vor Gebrauch hergestellt werden können. Die pharmazeutischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier-, Dragier-, Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Die Dosierung hängt von der Applikationsweise, der Spezies, dem Alter und von dem individuellen Zustand ab. Die täglichen Dosen der freien Basen oder von pharmazeutisch annehmbaren Salzen und derselben bewegen sich zwischen 0,01 mg/kg und 0,5 mg/kg für Warmblüter im allgemeinen und 0,001 g bis 0,01 g für Warmblüter mit einem Gewicht von etwa 70 kg.

Die folgenden Beispiele dienen zur Illustration der Erfindung; Temperaturen werden in Celsiusgraden angegeben.

## Beispiel 1

Ein Gemisch von 18,5 g (0,05 Mol) 7-Brom-3-methyl-2,3,4,5-tetrahydro-1H-dibenz[2,3 : 6,7]oxepino[4,5-d]azepin und 5,35 g (0,06 Mol) Kupfer(I)-cyanid in 20 ml Dimethylformamid wird in einer Stickstoffatmosphäre 24 Stunden unter Rühren auf 180° erwärmt. Hierauf wird das Gemisch auf 30° abgekühlt, mit 100 ml Methylenchlorid verdünnt und mit 50 ml einer 50%igen wäßrigen Äthylendiaminlösung versetzt. Die organische Phase wird anschließend abgetrennt, mit Wasser gewaschen und nach dem Trocknen über Natriumsulfat eingedampft. Der kristalline Rückstand, das 7-Cyano-3-methyl-2,3,4,5-tetrahydro-1H-dibenz[2,3 : 6,7]oxepino[4,5-d]azepin schmilzt nach dem Umkristallisieren aus Aceton bei 181—183°.

Zur Überführung ins Metansulfonat löst man 12,1 g (0,04 Mol) davon in 250 ml Aceton und versetzt diese Lösung unter Rühren mit 3,84 g Methansulfonsäure, worauf das 7-Cyano-3-methyl-2,3,4,5-tetrahydro-1H-dibenz[2,3 : 6,7]oxepino[4,5-d]azepin-methansulfonat vom Smp. 265—268° auskristallisiert.

Das Ausgangsmaterial kann auf folgende Weise hergestellt werden:

Ein Gemisch von 560,0 g 2-(4-Brom-phenyloxy)-benzoesäure in 1940 ml absolutem Benzol, 218 ml absolutem Äthanol und 32,6 ml konzentrierter Schwefelsäure wird 32 Stunden unter Rückfluß gekocht, wobei man das sich bildende Wasser in einem Wasserabscheider entfernt. Man kühlt das Reaktionsgemisch auf 10° ab und wäscht unter Zusatz von Eis mit 1000 ml Wasser, 500 ml einer 2-n. wäßrigen Natriumcarbonatlösung und anschließend nochmals mit 1000 ml Wasser. Die organische Phase wird abgetrennt, über Magnesiumsulfat getrocknet und unter einem Druck von 1 mm Hg bei 40° eingedampft. Der Rückstand wird im Hochvakuum destilliert und ergibt den 2-(4-Bromphenyloxy)-benzoesäure-äthylester, Kp. 140—150°/0,05 mm Hg.

Eine Lösung von 477,0 g des 2-(4-Brom-phenyloxy)-benzoesäureäthylesters in 900 ml absolutem Diäthyläther wird innerhalb 1 Stunde und unter Durchleiten eines Stickstoffstroms zu 42,3 g Lithiumaluminiumhydrid in 500 ml Diäthyläther eingetropft. Das Reaktionsgemisch wird 6 Stunden unter Rückfluß gekocht, auf 0—5° abgekühlt und unter Durchleiten von Stickstoff mit 450 ml Essigsäureäthylester und anschließend vorsichtig mit 350 ml Wasser versetzt. Man filtriert vom Niederschlag ab und wäscht diesen mit Diäthyläther nach. Die wäßrige Phase des Filtrats wird abgetrennt und mit 100 ml Diäthyläther gewaschen; die vereinigten organischen Phasen werden über Magnesiumsulfat getrocknet und unter 11 mm Hg zur Trockne eingedampft; der 2-(4-Brom-phenyloxy)-benzylalkohol bleibt als farbloses Öl zurück.

Ein Gemisch von 413,0 g des 2-(4-Brom-phenyloxy)-benzylalkohols und 1290 ml 48%iger Bromwasserstoffsäure wird 4 Stunden unter Rückfluß gekocht, dann abgekühlt und auf 2000 ml Eis und Wasser ausgegossen. Das ausgeschiedene grünliche Öl wird in 2000 ml Diäthyläther gelöst. Die organische Phase wird zweimal mit 400 ml Wasser und mit 400 ml einer 1-n. wäßrigen Natriumhydrogencarbonatlösung gewaschen, über Magnesiumsulfat getrocknet und unter 11 mm Hg bei 40° eingedampft. Das als öliger Rückstand erhaltene 2-(4-Bromphenyloxy)-benzylbromid wird ohne Reinigung weiterverwendet.

Man fügt 457,8 g des rohen 2-(4-Brom-phenyloxy)-benzylbromids innerhalb einer Stunde zu einem unter Rückfluß siedenden Gemisch von 171,0 g Natriumcyanid in 160 ml Wasser und 44 ml Äthanol; gleichzeitig werden 362 ml Äthanol zugetropft. Anschließend kocht man 3 weitere Stunden unter Rückfluß und verdünnt dann mit 1500 ml Wasser. Man wäscht die wäßrig-äthanolische Phase mit 1000 ml Diäthyläther, trennt die Ätherphase ab, wäscht sie zweimal mit 200 ml Wasser, trocknet sie über Magnesiumsulfat und engt sie unter 11 mm Hg zur Trockne ein. Den Rückstand kristallisiert man aus einem Gemisch von Diäthyläther und Petroläther und erhält so das 2-(4-Bromphenyloxy)-phenylacetonitril, F. 56—58°.

25,3 g Natrium werden unter Rühren in 400 ml abs. Äthanol gelöst, und anschließend destilliert man wieder ca. 200 ml abs. Äthanol aus dem Reaktionsgemisch ab. Hierauf gibt man 1500 ml abs. Toluol zu und destilliert unter einer Vigreuxkolonne weiter, bis der Siedepunkt 108° erreicht hat, wobei das Natriumäthylat auskristallisiert. Bei 100—110° wird nun innerhalb einer Stunde ein Gemisch von 288 g (1 Mol) 2-(4-Bromphenyloxy)-phenylacetonitril und 354 g (3 Mol) Diäthylcarbonat unter gleichzeitigem Abdestillieren des entstehenden Äthanols zugetropft. Nach Beendigung des Zutropfens wird das Reaktionsgemisch weiter destilliert bis der Siedepunkt wieder 108—110° erreicht hat. Anschließend wird das Reaktionsgemisch auf Raumtemperatur abgekühlt, mit 300 ml abs. Toluol verdünnt und innerhalb einer Stunde 170 g (1,2 Mol) Methyljodid zugetropft. Zur vollständigen Umsetzung wird das Gemisch 1 Stunde bei Raumtemperatur und 5 Stunden bei 80° weitergeführt. Nach dem Abkühlen auf Raumtemperatur läßt man 1 Liter Wasser zulaufen, trennt die organische Phase ab, wäscht sie mit Wasser und dampft sie nach dem Trocknen über Natriumsulfat im Vakuum vollständig ein. Als Rückstand bleibt der rohe 2-(4-Bromphenyloxy)-phenyl-$\alpha$-methyl-cyanessigsäure-äthylester zurück.

Unter gutem Rühren werden 374 g (1 Mol) roher 2-(4-Bromphenyloxy)-phenyl-$\alpha$-methyl-cyanessigsäure-äthylester, 830 ml Äthanol 96%ig und 460 ml 50%ige wäßrige Kalilauge 24 Stunden unter Rückfluß gekocht. Dann wird das Reaktionsgemisch unter 11 mm Hg bei 50° eingedampft und der Rückstand in 3500 ml Wasser gelöst. Nach dem Klarfiltrieren wird die alkalische Lösung mit konzentrierter Salzsäure angesäuert, wobei die 2-(4-Bromphenyloxy)-phenyl-$\alpha$-methylessigsäure auskristallisiert. Nach dem Abfiltrieren wird die erhaltene Säure im Vakuum bei 50° getrocknet und anschließend aus Acetonitril umkristallisiert, Smp. 99—101°.

321 g (1 Mol) 2-(4-Bromphenyloxy)-phenyl-$\alpha$-methylessigsäure und 3210 g Polyphosphorsäure werden unter gutem Rühren eine Stunde auf 100—105° erwärmt. Anschließend gießt man das Reaktionsgemisch unter Rühren in 3 Liter Wasser, wobei durch Eiszugabe die Temperatur unter 10° gehalten wird. Das ausgeschiedene Öl wird mit Diäthyläther extrahiert, die organische Phase wird mit Wasser gewaschen, über Kaliumcarbonat getrocknet und eingeengt, wobei nach dem Abkühlen das 8-Brom-11-methyl-dibenz[b,f]oxepin-10(11H)-on vom Smp. 85—87° auskristallisiert.

Zu einer Grignardlösung, die aus 49 g (2 Mol) Magnesium, 450 ml abs. Äther und 284 g Methyljodid bereitet wird, läßt man innerhalb 5 Stunden unter gutem Rühren eine Lösung von 303 g (1 Mol) 8-Brom-11-methyl-dibenz[b,f]oxepin-10(11H)-on in 1500 ml abs. Toluol eintropfen, wobei man eine Reaktionstemperatur von −5 bis 5° einhält. Anschließend erwärmt man das Reaktionsgemisch auf 55° und rührt es 15 Stunden bei dieser Temperatur weiter. Hierauf kühlt man das Reaktionsgemisch auf 0° ab und gießt es unter Rühren auf eine Lösung von 680 g Ammoniumchlorid in 2000 ml Eiswasser. Die organische Phase wird abgetrennt und die wäßrige Phase mit Toluol extrahiert. Die vereinigten organischen Lösungen wäscht man mit Wasser, trocknet sie über Natriumsulfat und dampft sie im Vakuum ein. Als Rückstand bleibt das 8-Brom-10,11-dimethyl-dihydrodibenz[b,f]oxepin-10-ol als Öl zurück.

319 g (1 Mol) 8-Brom-10,11-dimethyl-dihydro-dibenz[b,f]oxepin-10-ol (Rohprodukt) und 1,5 g p-Toluolsulfonsäure werden in einer Destillationsapparatur unter 11 mm Hg 1 Stunde auf 180° und 5 Stunden auf 200° Außentemperatur erhitzt, wobei sich Wasser abspaltet. Anschließend wird die Destillationsvorlage ausgewechselt und das entstandene 2-Brom-10,11-dimethyl-dibenz[b,f]oxepin im Hochvakuum destilliert, Kp. 142—148°/0,01 Torr. Das hellgelbe Destillat löst man in 300 ml Acetonitril und kühlt die Lösung auf 0° ab, wobei das Produkt auskristallisiert, Smp. 117—119°.

30,1 g (0,1 Mol) 2-Brom-10,11-dimethyl-dibenz[b,f]oxepin, werden in 525 ml Tetrachlorkohlenstoff gelöst, und diese Lösung wird mit 35,6 g (0,2 Mol) N-Bromsuccinimid versetzt. Unter Rühren in einer Stickstoffatmosphäre wird unter Belichten mit einer UV-Lampe das Gemisch zum Sieden erhitzt. Man erhält das Gemisch so lange am Sieden, bis alles N-Bromsuccinimid, welches auf dem Boden des Gefäßes liegt, sich in auf der Lösung schwimmendes Succinimid umgewandelt hat; Dauer etwa 10 Minuten. Hierauf kühlt man das Reaktionsgemisch auf 20° ab und trennt das Succinimid durch Filtration ab. Das Filtrat wird mit Wasser gewaschen, über Natriumsulfat getrocknet und im Vakuum eingeengt. Beim Abkühlen kristallisiert das 2-Brom-10,11-bis-brommethyl-dibenz[b,f]oxepin vom Smp. 124—126°.

45,9 g (0,1 Mol) 2-Brom-10,11-bis-(brommethyl)-dibenz[b,f]oxepin werden unter Rühren in einer Stickstoffatmosphäre in 500 ml Acetonitril suspendiert. Zu dieser Suspension wird innerhalb von 10 Minuten eine Lösung von 11,8 g (0,24 Mol) Natriumcyanid in 36 ml destilliertem Wasser getropft. Anschließend rührt man 1$^1/_2$ Stunden weiter, wobei die Innentemperatur durch schwaches Kühlen bei 20° gehalten wird. Nach dieser Zeit ist alles Ausgangsmaterial umgesetzt, und eine braune Lösung ist entstanden. Diese Reaktionslösung wird mit Wasser gewaschen und im Vakuum eingeengt, wobei das 2-Brom-dibenz[b,f]oxepin-10,11-diacetonitril vom Smp. 241—243° auskristallisiert.

35,1 g (0,1 Mol) 2-Brom-dibenz[b,f]oxepin-10,11-diacetonitril werden unter Rühren in 400 ml Methanol und 3,6 ml Wasser suspendiert und in einem Eisbad auf 0—5° abgekühlt. Hierauf wird bis zur Sättigung trockener Chlorwasserstoff eingeleitet. Das Reaktionsgemisch wird 12 Stunden bei 20° weitergerührt und anschließend 24 Stunden am Rückfluß gekocht. Anschließend dampft man die Reaktionslösung im Vakuum ein und extrahiert den Rückstand mit Äther. Die organische Phase wird abgetrennt, mit Wasser und 2 N-Sodalösung gewaschen, über Natriumsulfat getrocknet und eingedampft. Der rohe 2-Brom-dibenz[b,f]oxepin-10,11-diessigsäuremethylester bleibt als gelbes Öl zurück.

In eine eisgekühlte Lösung von 40 g (0,3 Mol) wasserfreiem Aluminiumchlorid in 1 Liter absolutem Diäthyläther läßt man unter Rühren und Feuchtigkeitsausschluß in einer Stickstoffatmosphäre langsam eine Suspension von 11,4 g (0,3 Mol) Lithiumaluminiumhydrid in 750 ml absolutem Diäthyläther derart zulaufen, daß die Reaktionstemperatur nicht über 5° steigt. Anschließend läßt man bei −2° bis +3° innerhalb 30 Minuten eine Lösung von 41,7 g (0,1 Mol) rohem 2-Brom-dibenz[b,f]oxepin-10,11-diessigsäuremethylester in 300 ml absolutem Diäthyläther zutropfen. Nach Beendigung des Zutropfens wird das Reaktionsgemisch noch 17 Stunden bei Raumtemperatur weitergerührt, dann auf 0—5° abgekühlt und das überschüssige Aluminiumhydrid vorsichtig durch tropfenweise Zugabe von 100 ml Wasser zersetzt. Hierauf wird die organische Phase abgetrennt, zweimal mit je 300 ml Wasser gewaschen, über Natriumsulfat getrocknet und eingedampft. Als Rückstand bleibt das rohe 2-Brom-dibenz[b,f]oxepin-10,11-diäthanol als gelbes Öl zurück.

36,1 g (0,1 Mol) rohes 2-Brom-dibenz[b,f]oxepin-10,11-diäthanol werden bei Raumtemperatur in 120 ml Pyridin gelöst und unter Rühren in einem Eis-Natriumchloridbad bei einer Reaktionstemperatur von −5° tropfenweise mit 25,2 g (0,22 Mol) Methansulfochlorid versetzt. Anschließend rührt man das Reaktionsgemisch 30 Minuten bei 0° und zwei Stunden bei 15—25°. Danach wird es zusammen mit Methylenchlorid in einem Scheidetrichter hintereinander mit je 800 ml 2-n. Salzsäure und Wasser gewaschen, über Natriumsulfat getrocknet und im Vakuum eingedampft. Als Rückstand verbleibt das rohe 2-Brom-10,11-bis-[2-(methylsulfonyloxy)äthyl]-dibenz[b,f]oxepin als hellbraunes Öl zurück.

51,7 g (0,1 Mol) rohes 2-Brom-10,11-bis-[2-(methylsulfonyloxy)-äthyl]-dibenz[b,f]oxepin werden mit einer Lösung von 60 g (1,36 Mol) Methylamin in 350 ml Äthanol 3 Stunden bei einer Innentemperatur von 60° gerührt, wobei sich nach 30 Minuten langsam Kristalle abscheiden. Anschließend wird das Reaktionsgemisch in einem Eisbad abgekühlt, die ausgefallenen Kristalle abgenutscht und mit Äthanol gewaschen. Das Nutschgut, das 7-Brom-3-methyl-2,3,4,5-tetrahydro-1H-dibenz[2,3 : 6,7]oxepino[4,5-d]azepin, schmilzt nach dem Trocknen bei 80° im Vakuumschrank bei 189—192°.

Beispiel 2

Analog Beispiel 1 wird aus 20,6 g (0,05 Mol) 7-Brom-3-(cyclopentylmethyl)-2,3,4,5-tetrahydro-1H-dibenz[2,3 : 6,7]oxepino[4,5-d]azepin und 5,35 g (0,06 Mol) Kupfer(I)-cyanid in 20 ml Dimethylformamid das 7-Cyano-3-(cyclopentylmethyl)-2,3,4,5-tetrahydro-1H-dibenz[2,3 : 6,7]oxepino[4,5-d]azepin, vom Smp. 99—102° (aus Acetonitril) erhalten. Das Methansulfonat schmilzt bei 266—269° (aus Äthanol).

Der Ausgangsstoff, das 7-Brom-3-(cyclopentylmethyl)-2,3,4,5-tetrahydro-1H-dibenz[2,3 : 6,7]oxepino[4,5-d]azepin, wird analog zum letzten Abschnitt von Beispiel 1 aus 51,7 g (0,1 Mol) rohem 2-Brom-10,11-bis-[2-(methylsulfonyloxy)-äthyl]-dibenz[b,f]oxepin und 29,7 g (0,3 Mol) (Aminomethyl)-cyclopentan in 100 ml Äthanol hergestellt, Smp. 125—218° (aus Acetonitril).

Beispiel 3

Zu einer gerührten Lösung von 6,0 g (0,02 Mol) 7-Cyano-3-methyl-2,3,4,5-tetrahydro-1H-dibenz[2,3 : 6,7]oxepino[4,5-d]azepin in 100 ml absolutem Benzol gibt man 0,2 g wasserfreies Kaliumcarbonat und tropft anschließend eine Lösung von 5,1 g (0,024 Mol) Chlorameisensäuretrichloräthylester in 20 ml absolutem Benzol bei einer Temperatur von 20 bis 25° zu. Das Reaktionsgemisch läßt man während 16 Stunden bei Raumtemperatur nachrühren und versetzt es hierauf mit 20 ml 5%igem Ammoniak. Die organische Phase wird abgetrennt, mit 5%iger wäßriger Methansulfonsäure gewaschen und nach dem Trocknen über Natriumsulfat auf ein kleines Volumen eingeengt, worauf der 7-Cyano-2,3,4,5-tetrahydro-1H-dibenz[2,3 : 6,7]oxepino[4,5-d]azepin-3-carbonsäure-trichloräthylester vom Smp. 178—180° auskristallisiert. 4,6 g (0,01 Mol) 7-Cyano-2,3,4,5-tetrahydro-1H-dibenz[2,3 : 6,7]oxepino[4,5-d]azepin-3-carbonsäure-trichloräthylester werden in 700 ml 90%iger Essigsäure gelöst und bei 35° auf einmal 6,8 g Zinkstaub zugegeben. Dieses Gemisch läßt man 6 Stunden bei Raumtemperatur rühren und dampft es anschließend am Rotationsverdampfer vollständig ein. Den Rückstand versetzt man unter Eiskühlung mit 100 ml konz. Ammoniak und extrahiert die ausgefallene Base mit Methylenchlorid. Die organische Phase wird abgetrennt, mit Wasser gewaschen, über Natriumsulfat getrocknet und eingedampft. Den öligen Rückstand löst man in 10 ml Äthanol und neutralisiert diese Lösung mit Methansulfonsäure, worauf das 7-Cyano-2,3,4,5-tetrahydro-1H-dibenz[2,3 : 6,7]oxepino[4,5-d]azepin-methansulfonat auskristallisiert, welches nach dem Umkristallisieren aus Methanol bei 219—222° schmilzt.

Beispiel 4

Tabletten, enthaltend 0,002 g 7-Cyano-3-methyl-2,3,4,5-tetrahydro-1H-dibenz[2,3 : 6,7]oxepino[4,5-d]azepin-methansulfonat werden, wie folgt hergestellt:

Zusammensetzung (für 10 000 Tabletten):

| | |
|---|---|
| 7-Cyano-3-methyl-2,3,4,5-tetrahydro-1H-dibenz-[2,3 : 6,7]oxepino[4,5-d]azepin-methansulfonat | 20,00 g |
| Lactose | 380,80 g |
| Kartoffelstärke | 354,70 g |
| Stearinsäure | 10,00 g |
| Talk | 200,00 g |
| Magnesiumstearat | 2,50 g |
| Kolloidales Siliciumdioxid | 32,00 g |
| Äthanol | q.s. |

Ein Gemisch des 7-Cyano-3-methyl-2,3,4,5-tetrahydro-1H-dibenz[2,3 : 6,7]oxepino[4,5-d]azepin-methansulfonat, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und preßt die Mischung zu Tabletten von je 0,1 g Gewicht, die gewünschtenfalls mit Teilkerben zur feineren Anpassung der Dosierung versehen sein können.

## Beispiel 5

Dragées, enthaltend 0,005 g des Methansulfonsäuresalzes des 7-Cyano-3-(cyclopentylmethyl)-2,3,4,5-tetrahydro-1H-dibenz[2,3 : 6,7]oxepino[4,5-d]azepin werden wie folgt hergestellt:

Zusammensetzung (für 10 000 Dragées):

| | |
|---|---|
| Methansulfonsäuresalz des 7-Cyano-3-(cyclopentylmethyl)-2,3,4,5-tetrahydro-1H-dibenz[2,3 : 6,7]oxepino[4,5-d]azepin | 50,00 g |
| Lactose | 175,90 g |
| Stearinsäure | 10,00 g |
| kolloidales Siliciumdioxid | 56,60 g |
| Talk | 165,00 g |
| Kartoffelstärke | 20,00 g |
| Magnesiumstearat | 2,50 g |
| Saccharose (krist.) | 502,28 g |
| Schellack | 6,00 g |
| arabische Gummi | 10,00 g |
| Farbstoff | 0,22 g |
| Titandioxid | 1,50 g |
| Äthanol | q.s. |

Aus dem 7-Cyano-3-(cyclopentylmethyl)-2,3,4,5-tetrahydro-1H-dibenz[2,3 : 6,7]oxepino[4,5-d]azepin-methansulfonat, der Lactose und einer äthanolischen Lösung der Stearinsäure stellt man ein Granulat her, das man nach dem Trocknen mit dem kolloidalen Siliciumdioxid, dem Talk, der Kartoffelstärke und dem Magnesiumstearat vermischt und zu Dragée-Kernen preßt. Diese werden anschließend mit einem konzentrierten Sirup aus der Saccharose, dem Schellack, dem arabischen Gummi, dem Farbstoff und dem Titandioxid überzogen und getrocknet. Man erhält so Dragées mit einem Gewicht von je 0,100 g enthaltend je 0,005 g Wirkstoff.

## Beispiel 6

Kapseln, enthaltend 0,002 g des 7-Cyano-3-methyl-2,3,4,5-tetrahydro-1H-dibenz[2,3 : 6,7]oxepino[4,5-d]azepin-methansulfonat werden wie folgt hergestellt:

Zusammensetzung (für 1000 Kapseln):

| | |
|---|---|
| 7-Cyano-3-methyl-2,3,4,5-tetrahydro-1H-dibenz [2,3 : 6,7]oxepino[4,5-d]azepin-methansulfonat | 2,00 g |
| Lactose | 271,00 g |
| Gelatine | 2,00 g |
| Maisstärke | 10,00 g |
| Talk | 15,00 g |
| Wasser | q.s. |

Man mischt das 7-Cyano-3-methyl-2,3,4,5-tetrahydro-1H-dibenz[2,3 : 6,7]oxepino[4,5-d]azepin-methansulfonat mit der Lactose, befeuchtet die Mischung gleichmäßig mit einer wäßrigen Lösung der

Gelatine und granuliert sie durch ein geeignetes Sieb (z. B. ein Sieb mit lichter Maschenweite von 1,2—1,5 mm. Das Granulat mischt man mit der getrockneten Maisstärke und dem Talk und füllt es gleichmäßig in die Hartgelatinekapseln (Größe 1) ab.

Beispiel 7

Eine wäßrige Injektionslösung, enthaltend 0,001 g/ml 7-Cyano-3-methyl-2,3,4,5-tetrahydro-1H-dibenz[2,3 : 6,7]oxepino[4,5-d]azepin-methansulfonat wird wie folgt hergestellt:

Zusammensetzung (für 1000 Ampullen):

| | |
|---|---|
| 7-Cyano-3-methyl-2,3,4,5-tetrahydro-1H-dibenz-[2,3 : 6,7]oxepino[4,5-d]azepin-methansulfonat | 1,00 g |
| Wasser | q.s. |

Eine Lösung des 7-Cyano-3-methyl-2,3,4,5-tetrahydro-1H-dibenz[2,3 : 6,7]oxepino[4,5-d]azepin-methansulfonat in 1000 ml Wasser wird in Ampullen abgefüllt und sterilisiert. Eine Ampulle enthält eine 0,1%ige Lösung des Wirkstoffes.

**Patentansprüche für die Vertragsstaaten: BE, CH, DE, FR, IT, NL, LU, LI, SE**

1. Azatetracyclische Carbonitrile der Formel

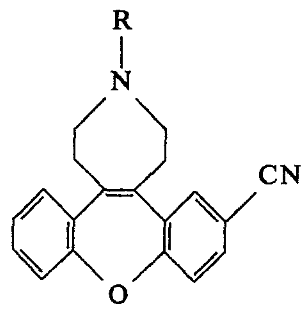

(I)

in welcher
R Wasserstoff, Niederalkyl mit bis zu 8 Kohlenstoffatomen, Cycloalkylniederalkyl mit bis zu 10 Kohlenstoffatomen, Allyl, 2-Methallyl, Propargyl, Hydroxyniederalkyl mit 2 bis 8 Kohlenstoffatomen, Niederalkoxyniederalkyl mit 3 bis 10 Kohlenstoffatomen oder Alkanoyloxyniederalkyl mit 3 bis 21 Kohlenstoffatomen bedeutet, wobei ein allfällig vorhandenes Sauerstoffatom durch mindestens 2 Kohlenstoffatome vom Ringstickstoffatom getrennt ist, sowie Säureadditionssalze von solchen Verbindungen.

2. Verbindungen der im Anspruch 1 angegebenen Formel I, worin R Wasserstoff, Niederalkyl mit bis 4 Kohlenstoffatomen, Cycloalkylniederalkyl mit 4—8 Kohlenstoffatomen, Allyl, Propargyl, Hydroxyniederalkyl mit 2 bis 4 Kohlenstoffatomen, Niederalkoxyniederalkyl mit 3 bis 6 Kohlenstoffatomen oder Alkanoyloxy-niederalkyl mit 4 bis 11 Kohlenstoffatomen bedeuten.

3. Verbindungen der im Anspruch 1 angegebenen Formel I, worin R Niederalkyl mit bis 4 Kohlenstoffatomen oder Cyclopentylmethyl bedeutet.

4. 7-Cyano-3-methyl-2,3,4,5-tetrahydro-1H-dibenzo[2,3 : 6,7]oxepino[4,5-d]azepin sowie Säureadditionssalze dieser Verbindung.

5. 3-(Cyclopentylmethyl)-7-cyano-2,3,4,5-tetrahydro-1H-dibenzo[2,3 : 6,7]oxepino[4,5-d]azepin sowie Säureadditionssalze dieser Verbindung.

6. Die pharmazeutisch annehmbaren Säureadditionssalze der Verbindungen gemäß den Ansprüchen 1—5.

7. Verfahren zur Herstellung von azatetracyclischen Carbonitrilen der Formel

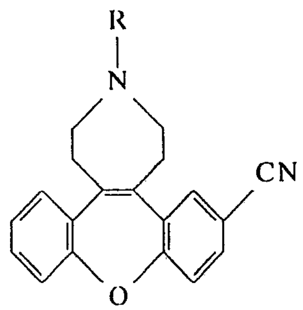

(I)

in welcher
R Wasserstoff, Niederalkyl mit bis zu 8 Kohlenstoffatomen, Cycloalkylniederalkyl mit bis zu 10 Kohlenstoffatomen, Allyl, 2-Methallyl, Propargyl, Hydroxyniederalkyl mit 2 bis 8 Kohlenstoffatomen, Niederalkoxyniederalkyl mit 3 bis 10 Kohlenstoffatomen oder Alkanoyloxyniederalkyl mit 3 bis 21 Kohlenstoffatomen bedeutet, wobei ein allfällig vorhandenes Sauerstoffatom durch mindestens 2 Kohlenstoffatome vom Ringstickstoffatom getrennt ist, sowie Säureadditionssalze von solchen Verbindungen, dadurch gekennzeichnet, daß man

a) einen reaktionsfähigen Diester des Diäthanols der Formel

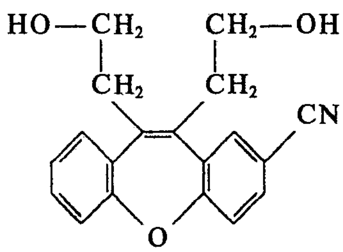

(II)

mit einer Verbindung der Formel

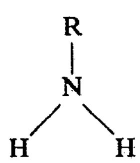

(III)

umsetzt oder

b) eine Verbindung der Formel

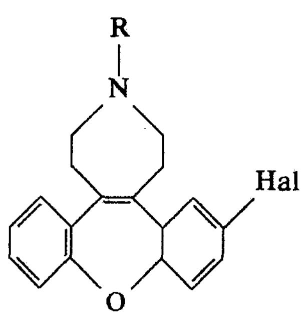

(IV)

in welcher Hal ein Halogenatom darstellt, mit einer Cyanidverbindung umsetzt und, wenn erwünscht, eine verfahrensgemäß erhaltene Verbindung der Formel I in eine andere Verbindung der Formel I umwandelt und/oder, wenn erwünscht, ein verfahrensgemäß erhaltenes Salz in die freie Verbindung oder in ein anderes Salz oder eine verfahrensgemäß erhaltene freie Verbindung in ein Salz umwandelt und/oder, wenn erwünscht, ein verfahrensgemäß erhaltenes Racemat in die optischen Antipoden trennt.

8. Pharmazeutische Zusammensetzungen, gekennzeichnet durch einen Gehalt an einer Verbindung gemäß Anspruch 1 oder einem pharmazeutisch annehmbaren Säureadditionssalz derselben und mindestens einem pharmazeutischen Trägerstoff.

9. Pharmazeutische Zusammensetzungen, gekennzeichnet durch einen Gehalt an einer Verbindung gemäß einem der Ansprüche 2 oder 3 oder einem pharmazeutisch annehmbaren Säureadditionssalz

derselben und mindestens einem pharmazeutischen Trägerstoff.

10. Pharmazeutische Zusammensetzungen, gekennzeichnet durch einen Gehalt an 3-(Cyclopentylmethyl)-7-cyano-2,3,4,5-tetrahydro-1H-dibenzo[2,3 : 6,7]oxepino[4,5-d]azepin oder einem pharmazeutisch annehmbaren Säureadditionssalz derselben und mindestens einem pharmazeutischen Trägerstoff.

11. Azatetracyclische Carbonitrile nach Anspruch 1 zur Anwendung in einem Verfahren zur therapeutischen Behandlung des menschlichen oder tierischen Körpers.

12. Azatetracyclische Carbonitrile nach einem der Ansprüche 2—6 zur Anwendung in einem Verfahren zur therapeutischen Behandlung des menschlichen oder tierischen Körpers.

13. 7-Cyano-3-methyl-2,3,4,5-tetrahydro-1H-dibenzo[2,3 : 6,7]oxepino[4,5-d]azepin zur Anwendung in einem Verfahren zur therapeutischen Behandlung des menschlichen oder tierischen Körpers.

14. 3-(Cyclopentylmethyl)-7-cyano-2,3,4,5-tetrahydro-1H-dibenzo[2,3 : 6,7]oxepino[4,5-d]azepin zur Anwendung in einem Verfahren zur therapeutischen Behandlung des menschlichen oder tierischen Körpers.

**Patentansprüche für den Vertragsstaat: AT**

1. Verfahren zur Herstellung von azatetracyclischen Carbonitrilen der Formel

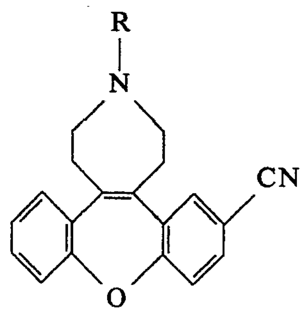

(I)

in welcher
R Wasserstoff, Niederalkyl mit bis zu 8 Kohlenstoffatomen, Cycloalkylniederalkyl mit bis zu 10 Kohlenstoffatomen, Allyl, 2-Methallyl, Propargyl, Hydroxyniederalkyl mit 2 bis 8 Kohlenstoffatomen, Niederalkoxyniederalkyl mit 3 bis 10 Kohlenstoffatomen oder Alkanoyloxyniederalkyl mit 3 bis 21 Kohlenstoffatomen bedeutet, wobei ein allfällig vorhandenes Sauerstoffatom durch mindestens 2 Kohlenstoffatome vom Ringstickstoffatom getrennt ist, sowie Säureadditionssalze von solchen Verbindungen, dadurch gekennzeichnet, daß man

a) einen reaktionsfähigen Diester des Diäthanols der Formel

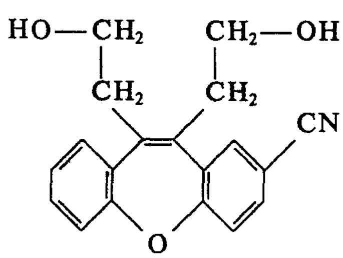

(II)

mit einer Verbindung der Formel

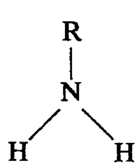

(III)

umsetzt oder

b) eine Verbindung der Formel

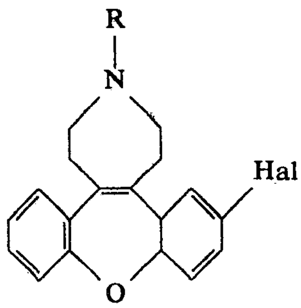

(IV)

in welcher Hal ein Halogenatom darstellt, mit einer Cyanidverbindung umsetzt und, wenn erwünscht, eine verfahrensgemäß erhaltene Verbindung der Formel I in eine andere Verbindung der Formel I umwandelt und/oder, wenn erwünscht, ein verfahrensgemäß erhaltenes Salz in die freie Verbindung oder in ein anderes Salz oder eine verfahrensgemäß erhaltene freie Verbindung in ein Salz umwandelt und/oder, wenn erwünscht, ein verfahrensgemäß erhaltenes Racemat in die optischen Antipoden trennt.

2. Verfahren nach Anspruch 1, dadurch gekennzeichnet, daß man Verbindungen der im Anspruch 1 angegebenen Formel I, worin R Wasserstoff, Niederalkyl mit bis 4 Kohlenstoffatomen, Cycloalkylniederalkyl mit 4 bis 8 Kohlenstoffatomen, Allyl, Propargyl, Hydroxyniederalkyl mit 2 bis 4 Kohlenstoffatomen, Niederalkoxyniederalkyl mit 3 bis 6 Kohlenstoffatomen oder Alkanoyloxy-niederalkyl mit 4 bis 11 Kohlenstoffatomen bedeuten, herstellt.

3. Verfahren nach Anspruch 1, dadurch gekennzeichnet, daß man Verbindungen der im Anspruch 1 angegebenen Formel I, worin R Niederalkyl mit bis 4 Kohlenstoffatomen oder Cyclopentylmethyl bedeutet, herstellt.

4. Verfahren nach Anspruch 1, dadurch gekennzeichnet, daß man 7-Cyano-3-methyl-2,3,4,5-tetrahydro-1H-dibenzo[2,3 : 6,7]oxepino[4,5-d]azepin, sowie Säureadditionssalze dieser Verbindung, herstellt.

5. Verfahren nach Anspruch 1, dadurch gekennzeichnet, daß man 3-(Cyclopentylmethyl)-7-cyano-2,3,4,5-tetrahydro-1H-dibenzo[2,3 : 6,7]oxepino[4,5-d]azepin, sowie Säureadditionssalze dieser Verbindung, herstellt.

6. Verfahren nach Anspruch 1, dadurch gekennzeichnet, daß man die pharmazeutisch annehmbaren Säureadditionssalze der in den Ansprüchen 1—5 angegebenen Verbindungen herstellt.

**Claims for the Contracting States: BE, CH, DE, FR, IT, NL, LU, LI, SE**

1. Azatetracyclic carbonitriles of the formula

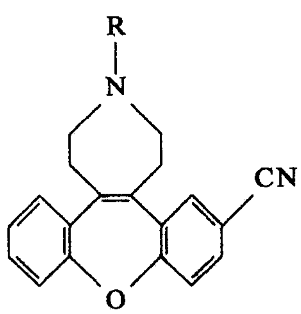

(I)

in which
R is hydrogen, lower alkyl having up to 8 carbon atoms, cycloalkyl-lower alkyl having up to 10 carbon atoms, allyl, 2-methallyl, propargyl, hydroxy-lower alkyl having 2 to 8 carbon atoms, lower-alkoxy-lower alkyl having 3 to 10 carbon atoms or alkanoyloxy-lower alkyl having 3 to 21 carbon atoms, any oxygen atom present being separated by at least 2 carbon atoms from the ring nitrogen atom; and acid addition salts of such compounds.

2. Compounds of the formula I given in claim 1, wherein R is hydrogen, lower alkyl having up to 4 carbon atoms, cycloalkyl-lower alkyl having 4—8 carbon atoms, allyl, propargyl, hydroxy-lower alkyl having 2 to 4 carbon atoms, lower-alkoxy-lower alkyl having 3 to 6 carbon atoms or alkanoyloxy-lower alkyl having 4 to 11 carbon atoms.

3. Compounds of the formula I given in claim 1, wherein R is lower alkyl having up to 4 carbon atoms, or cyclopentylmethyl.

4. 7-Cyano-3-methyl-2,3,4,5-tetrahydro-1H-dibenzo[2,3 : 6,7]oxepino[4,5-d]azepine and acid addition salts of this compound.

5. 3-(Cyclopentylmethyl)-7-cyano-2,3,4,5-tetrahydro-1H-dibenzo[2,3 : 6,7]oxepino[4,5-d]azepine and acid addition salts of this compound.

6. The pharmaceutically acceptable acid addition salts of the compounds according to claims 1 – 5.

7. Process for the production of azatetracyclic carbonitriles of the formula

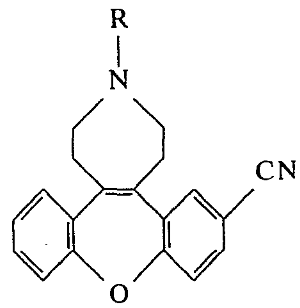

(I)

in which
R is hydrogen, lower alkyl having up to 8 carbon atoms, cycloalkyl-lower alkyl having up to 10 carbon atoms, allyl, 2-methallyl, propargyl, hydroxy-lower alkyl having 2 to 8 carbon atoms, lower-alkoxy-lower alkyl having 3 to 10 carbon atoms or alkanoyloxy-lower alkyl having 3 to 21 carbon atoms, any oxygen atom present being separated by at least 2 carbon atoms from the ring nitrogen atom; and acid addition salts of such compounds, characterised in that

a) a reactive diester of the diethanol of the formula

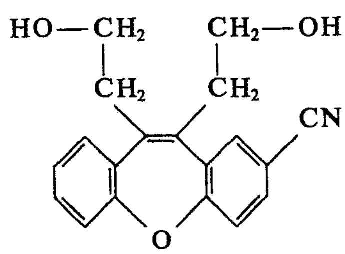

(II)

is reacted with a compound of the formula

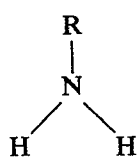

(III)

or

b) a compound of the formula

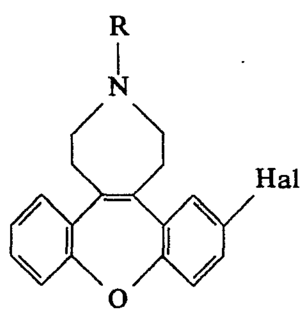

(IV)

in which Hal is a halogen atom, is reacted with a cyanide compound and, if desired, a compound of the formula I obtained according to the process is converted into a different compound of the formula I and/or, if desired, a salt obtained according to the process is converted into the free compound or into a different salt, or a free compound obtained according to the process is converted into a salt and/or, if desired, a racemate obtained according to the process is separated into the optical antipodes.

8. Pharmaceutical compositions, characterised in that they contain a compound according to claim 1 or a pharmaceutically acceptable acid addition salt thereof and at least one pharmaceutical carrier.

9. Pharmaceutical compositions, characterised in that they contain a compound according to one of claims 2 or 3, or a pharmaceutically acceptable acid addition salt thereof and at least one pharmaceutical carrier.

10. Pharmaceutical compositions, characterised in that they contain 3-(cyclopentylmethyl)-7-cyano-2,3,4,5-tetrahydro-1H-dibenzo[2,3 : 6,7]oxepino[4,5-d]azepine or a pharmaceutically acceptable acid addition salt thereof and at least one pharmaceutical carrier.

11. Azatetracyclic carbonitriles according to claim 1 for use in a method for the therapeutic treatment of the human or animal body.

12. Azatetracyclic carbonitriles according to one of claims 2—6 for use in a method for the therapeutic treatment of the human or animal body.

13. 7-Cyano-3-methyl-2,3,4,5-tetrahydro-1H-dibenzo[2,3 : 6,7]oxepino[4,5-d]azepine for use in a method for the therapeutic treatment of the human or animal body.

14. 3-(Cyclopentylmethyl)-7-cyano-2,3,4,5-tetrahydro-1H-dibenzo[2,3 : 6,7]oxepino[4,5-d]azepine for use in a method for the therapeutic treatment of the human or animal body.

**Claims for the Contracting State: AT**

1. Process for the production of azatetracyclic carbonitriles of the formula

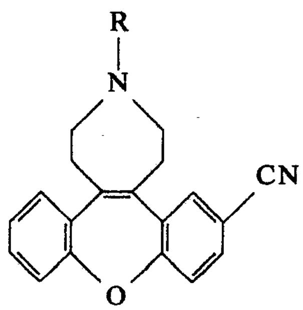

(I)

in which
R is hydrogen, lower alkyl having up to 8 carbon atoms, cycloalkyl-lower alkyl having up to 10 carbon atoms, allyl, 2-methallyl, propargyl, hydroxy-lower alkyl having 2 to 8 carbon atoms, lower-alkoxy-lower alkyl having 3 to 10 carbon atoms or alkanoyloxy-lower alkyl having 3 to 21 carbon atoms, any oxygen atom present being separated by at least 2 carbon atoms from the ring nitrogen atom; and acid addition salts of such compounds, characterised in that

a) a reactive diester of the diethanol of the formula

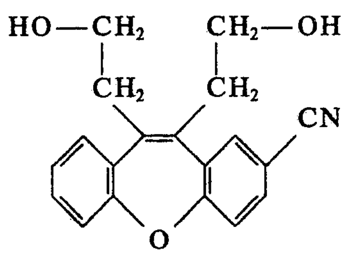

(II)

is reacted with a compound of the formula

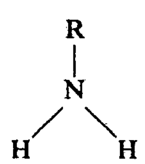

(III)

or

b) a compound of the formula

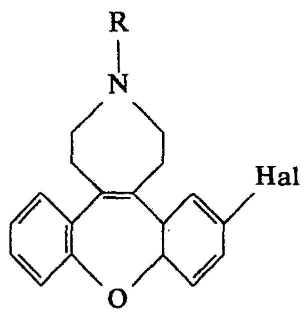

(IV)

in which Hal is a halogen atom, is reacted with a cyanide compound and, if desired, a compound of the formula I obtained according to the process is converted into a different compound of the formula I and/or, if desired, a salt obtained according to the process is converted into the free compound or into a different salt, or a free compound obtained according to the process is converted into a salt and/or, if desired, a racemate obtained according to the process is separated into the optical antipodes.

2. Process according to claim 1, characterised in that there are produced compounds of the formula I given in claim 1, wherein R is hydrogen, lower alkyl having up to 4 carbon atoms, cycloalkyl-lower alkyl having 4—8 carbon atoms, allyl, propargyl, hydroxy-lower alkyl having 2 to 4 carbon atoms, lower-alkoxy-lower alkyl having 3 to 6 carbonatoms or alkanoyloxy-lower alkyl having 4 to 11 carbon atoms.

3. Process according to claim 1, characterised in that there are produced compounds of the formula I given in claim 1, wherein R is lower alkyl having up to 4 carbon atoms, or cyclopentylmethyl.

4. Process according to claim 1, characterised in that 7-cyano-3-methyl-2,3,4,5-tetrahydro-1H-dibenzo[2,3 : 6,7]oxepino[4,5-d]azepine and acid addition salts of this compound are produced.

5. Process according to claim 1, characterised in that 3-(cyclopentylmethyl)-7-cyano-2,3,4,5-tetrahydro-1H-dibenzo[2,3 : 6,7]oxepino[4,5-d]azepine and acid addition salts of this compound are produced.

6. Process according to claim 1, characterised in that the pharmaceutically acceptable acid addition salts of the compounds given in claims 1—5 are produced.

**Revendications pour les Etats contractants: BE, CH, DE, FR, IT, NL, LU, LI, SE**

1. Carbonitriles azatétracycliques de formule

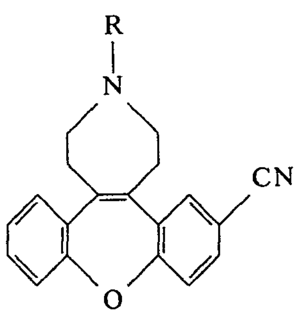

(I)

où

R représente un hydrogène, un alcoyle inférieur ayant jusqu'à 8 atomes de carbone, un cycloalcoyl-alcoyle inférieur ayant jusqu'à 10 atomes de carbone, un allyle, un 2-méthallyle, un propargyle, un hydroxy-alcoyle inférieur en $C_2$ à $C_8$, un alcoxy inférieur-alcoyle inférieur en $C_3$ à $C_{10}$ ou un alcanoyloxy-alcoyle inférieur en $C_3$ à $C_{21}$, où un atome d'oxygène éventuellement présent est séparé de l'atome d'azote du noyau par au moins deux atomes de carbone, ainsi que les sels d'addition d'acides de tels composés.

2. Composés de formule I donnée dans la revendications 1, où R représente un hydrogène, un alcoyle inférieur ayant jusqu'à 4 atomes de carbone, un cycloalcoyl-alcoyle inférieur en $C_4$ à $C_8$, un allyle, un propargyle, un hydroxyalcoyle inférieur en $C_2$ à $C_4$, un alcoxy inférieur-alcoyle inférieur en $C_3$ à $C_6$ ou un alcanoyloxy-alcoyle inférieur en $C_4$ à $C_{11}$.

3. Composés de formule I donnée dans la revendication 1, où R représente un alcoyle inférieur ayant jusqu'à 4 atomes de carbone ou un cyclopentylméthyle.

4. La 7-cyano-3-méthyl-2,3,4,5-tétrahydro-1H-dibenzo[2,3 : 6,7]oxépino[4,5-d]azépine, ainsi que les sels d'addition de ce composé.

5. La 3-(cyclopentylméthyl)-7-cyano-2,3,4,5-tétrahydro-1H-dibenzo[2,3 : 6,7]oxépino[4,5-d]azépine,

ainsi que les sels d'addition d'acides de ce composé.

6. Les sels d'addition d'acides pharmaceutiquement acceptables des composés selon les rev. 1—5.

7. Procédé de préparation de carbonitriles azatétracycliques de formule

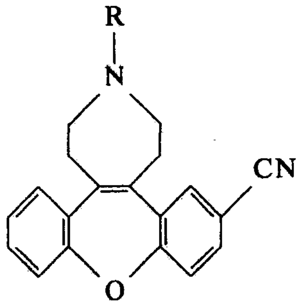

(I)

où

R représente un hydrogène, un alcoyle inférieur ayant jusqu'à 8 atomes de carbone, un cycloalcoyl-alcoyle inférieur ayant jusqu'à 10 atomes de carbone, un allyle, un 2-méthallyle, un propargyle, un hydroxy-alcoyle inférieur en $C_2$ à $C_8$, un alcoxy inférieur-alcoyle inférieur en $C_3$ à $C_{10}$ ou un alcanoyloxy-alcoyle inférieur en $C_3$ à $C_{21}$, où un atome d'oxygène éventuellement présent est séparé de l'atome d'azote du noyau par au moins deux atomes de carbone, ainsi que les sels d'addition d'acides de tels composés, caractérisé en ce que

a) on fait réagir und diester réactif du diéthanol de formule

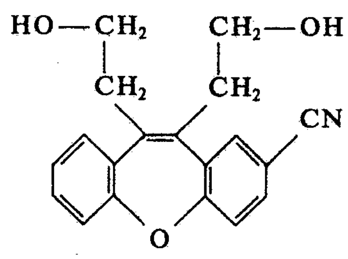

(II)

avec un composé de formule

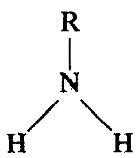

(III)

ou

b) on fait réagir un composé de formule

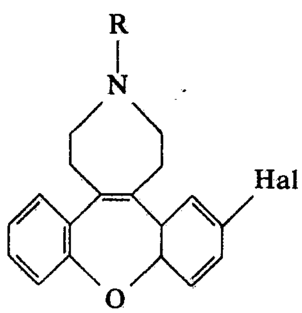

(IV)

où Hal représente un atome d'halogène, avec un composé de cyanure et, si on le désire, on transforme un composé de formule I obtenu selon l'invention, en un autre composé de formule I et/ou, si on le désire, on transforme un sel obtenu selon le procédé, en le composé libre ou en un autre sel, ou un composé libre obtenu selon le procédé en un sel et/ou, si on le désire, on dédouble un racémate obtenu selon lon procédé, pour donner les antipodes optiques.

8. Compositions pharmaceutiques caractérisées en ce qu'elles contiennent un composé selon la rev. 1 ou un de ses sels d'addition d'acide pharmaceutiquement acceptable et au moins un support pharmaceutique.

9. Compositions pharmaceutiques caractérisées en ce qu'elles contiennent un composé selon l'une des rev. 2 ou 3, ou un de ses sels d'addition d'acides pharmaceutiquement acceptables et au moins un support pharmaceutique.

10. Compositions pharmaceutiques caractérisées en ce qu'elles contiennent la 3-(cyclopentylméthyl)-7-cyano-2,3,4,5-tétrahydro-1H-dibenzo[2,3 : 6,7]oxépino[4,5-d]azépine, ou un de ses sels d'addition d'acides pharmaceutiquement acceptables et au moins un support pharmaceutique.

11. Carbonitriles azatétracycliques selon la rev. 1 aux fins d'application dans un procédé de traitement thérapeutique du corps humain ou animal.

12. Carbonitriles azatétracycliques selon l'une des rev. 2—6 aux fins d'application dans un procédé de traitement thérapeutique du corps humain ou animal.

13. La 7-cyano-3-méthyl-2,3,4,5-tétrahydro-1H-dibenzo[2,3 : 6,7]oxépino[4,5-d]azépine aux fins d'application dans un procédé de traitement thérapeutique du corps humain ou animal.

14. La 3-(cyclopentylméthyl)-7-cyano-2,3,4,5-tétrahydro-1H-dibenzo[2,3 : 6,7]oxépino[4,5-d]azépine aux fins d'application dans un procédé de traitment thérapeutique du corps humain ou animal.

**Revendications pour l'Etat contractant: AT**

1. Procédé de préparation de carbonitriles azatétracycliques de formule

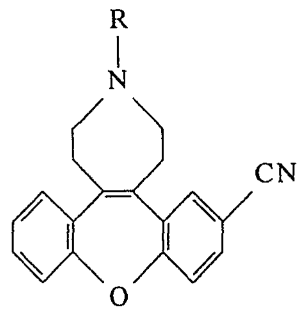

(I)

où

R représente un hydrogène, un alcoyle inférieur ayant jusqu'à 8 atomes de carbone, un cycloalcoyl-alcoyle inférieur ayant jusqu'à 10 atomes de carbone, un allyle, un 2-méthallyle, un propargyle, un hydroxy-alcoyle inférieur en $C_2$ à $C_8$, un alcoxy inférieur-alcoyle inférieur en $C_3$ à $C_{10}$ ou un alcanoyloxy-alcoyle inférieur en $C_3$ à $C_{21}$, où un atome d'oxygène éventuellement présent est séparé de l'atome d'azote du noyau par au moins deux atomes de carbone, ainsi que les sels d'addition d'acides de tels composés, caractérisé en ce que

a) on fait réagir und diester réactif du diéthanol de formule

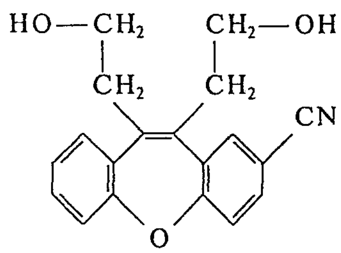

(II)

avec un composé de formule

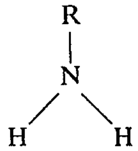

(III)

ou

b) on fait réagir un composé de formule

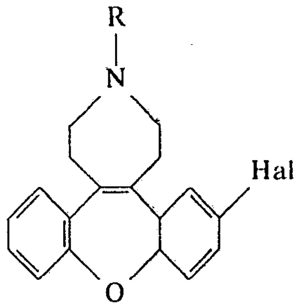

(IV)

où Hal représente un atome d'halogène, avec un composé de cyanure et, si on le désire, on transforme un composé de formule I obtenu selon l'invention, en un autre composé de formule I et/ou, si on le désire, on transforme un sel obtenu selon le procédé, en le composé libre ou en un autre sel, ou un composé libre obtenu selon le procédé en un sel et/ou, si on le désire, on dédouble un racémate obtenu selon le procédé, pour donner les antipodes optiques.

2. Procédé selon la rev. 1, caractérisé en ce qu'on prépare des composés de formule I donnés dans la rev. 1, où R est un atome d'hydrogène, un alcoyle inférieur en $C_1$ à $C_4$, un cycloalcoyl-alcoyle inférieur en $C_4$ à $C_8$, un allyle, un propargyl, un hydroalcoyle inférieur en $C_2$ à $C_4$, un alcoxy inférieur-alcoyle inférieur en $C_3$ à $C_6$ ou un alcanoyloxy-alcoyle inférieur en $C_4$ à $C_{11}$.

3. Procédé selon la rev. 1, caractérisé en ce qu'on prépare des composés de formule I données dans la rev. 1, où R représente un alcoyle inférieur ayant jusqu'à 4 atomes de carbone ou un cyclopentylméthyle.

4. Procédé selon la rev. 1, caractérisé en ce qu'on prépare la 7-cyano-3-méthyl-2,3,4,5-tétrahydro-1H-dibenzo[2,3 : 6,7]oxépino[4,5-d]azépine, ainsi que les sels d'addition d'acides de ces composés.

5. Procédé selon la rev. 1, caractérisé en ce qu'on prépare la 3-(cyclopentylméthyl)-7-cyano-2,3,4,5-tétrahydro-1H-dibenzo[2,3 : 6,7]oxépino[4,5-d]azépine, ainsi que les sels d'addition d'acides de ces composés.

6. Procédé selon la rev. 1, caractérisé en ce qu'on prépare les sels d'addition d'acides pharmaceutiquement acceptables des composés indiqués dans les rev. 1 à 5.